# FASCICULE DE BREVET EUROPEEN

(11) **EP 1 163 366 B1**
(45) Date de publication et mention de la délivrance du brevet: **08.08.2007**
(21) Numéro de dépôt: 00907713.2
(22) Date de dépôt: 24.02.2000
(51) Int. Cl.: C12Q 1/68, G06F 17/30

(54) **SELECTION RATIONNELLE DE PEPTIDES PUTATIFS EN PROVENANCE DE SEQUENCES NUCLEOTIDIQUES OU PEPTIDIQUES IDENTIFIEES**
RATIONALE AUSWAHL VON MUTMASSLICHEN PEPTIDEN AUS IDENTIFIZIERTEN NUKLEOTID- BEZIEHUNGSWEISE PEPTIDSEQUENZEN
RATIONAL SELECTION OF PEPTIDES PRESUMED TO HAVE A SPECIFIC FUNCTION DERIVED FROM IDENTIFIED NUCLEOTIDE OR PEPTIDE SEQUENCES

(30) Priorité: 25.02.1999 US 257525
(43) Date de publication de la demande: 19.12.2001
(73) Titulaire: SOCIETE DE CONSEILS DE RECHERCHES ET D'APPLICATIONS SCIENTIFIQUES (S.C.R.A.S.), 75016 Paris (FR); CENTRE NATIONAL DE LA RECHERCHE SCIENTIFIQUE (CNRS), 75794 Paris Cedex 16 (FR)
(72) Inventeur: CAMARA Y FERRER, José, Antonio, F-75016 Paris (FR); THURIEAU, Christophe, F-75116 Paris (FR); MARTINEZ, Jean, F-34570 Saussan (FR); BERGE, Gilbert, F-34090 Montpellier (FR); GOZE, Catherine, Résidence Château d'Alco, 34080 Montpellier (FR)
(74) Mandataire: Bourgouin, André
(86) Numéro de dépôt international: PCT/FR2000/000460
(87) Numéro de publication internationale: WO 2000/050636

(56) Documents cités:
- WO-A-99/10361
- US-A- 5 523 208
- LANG F: "SWISS-PROT + TREMBL" TRENDS IN GENETICS,NL,ELSEVIER SCIENCE PUBLISHERS B.V. AMSTERDAM, vol. 13, no. 10, 1 octobre 1997 (1997-10-01), page 417 XP004090564 ISSN: 0168-9525
- KERLAVAGE A R ET AL: "DATA MANAGEMENT AND ANALYSIS FOR HIGH-THROUGHPUT DNA SEQUENCING PROJECTS" IEEE ENGINEERING IN MEDICINE AND BIOLOGY MAGAZINE,US,IEEE INC. NEW YORK, vol. 14, no. 6, 1 novembre 1995 (1995-11-01), pages 710-717, XP000598295 ISSN: 0739-5175
- NEVILL-MANNING C.G. ET AL: 'Highly specific protein sequence motifs for genome analysis.' PROC. NATL. ACAD. SCI. USA vol. 95, 1998, pages 5865 - 5871
- BAIROCH A. ET AL: 'The SWISS-PROT protein sequence data bank and its supplement TrEMBL.' NUCLEIC ACIDS RES. vol. 25, no. 1, 1997, pages 31 - 36

## Description

### ART ANTERIEUR RELATIF A L'INVENTION :

### (i) Domaine de l'invention

La présente invention se rapporte à une méthode d'identification de peptides putatifs en provenance de séquences nucléotidiques ou peptidiques de fonction inconnue, comme par exemple à la fois des précurseurs d'acides nucléiques et de peptides comprenant une extrémité C-terminale amidée, et, plus particulièrement, à une méthode caractérisée en ce que les précurseurs de peptides putatifs sont identifiés à partir d'une banque de données génétique.

### (ii) Description de l'art antérieur apparenté

On sait que certaines combinaisons de nucléotides, quand elles sont présentes dans un polynucléotide, conduisent à certaines propriétés dans le polypeptide qui en est traduit. Un exemple comprend ces nucléotides qui encodent les précurseurs d'hormones polypeptidiques qui sont soumis à une réaction d'amidation post-traductionnelle. Un autre exemple, tel divulgué dans le brevet américain No. 4,917,999, se rapporte à certains nucléotides qui sont caractéristiques de polypeptides manifestant une activité enzymatique α-amylase.

Les hormones polypeptidiques amidées sont synthétisées sous la forme d'un précurseur qui subit une maturation. Cette maturation consiste en une réaction d'amidation. La réaction d'amidation de l'extrémité C-terminale est une réaction caractéristique des hormones polypeptidiques amidées. Cette réaction, qui se produit sur le précurseur d'une ou de plusieurs hormones, permet la maturation de l'hormone et garantit également sa stabilité biologique dans le milieu physiologique: le groupe amide formé est moins vulnérable que la fonction acide libre. L'hormone est, par conséquent, plus résistante aux carboxypeptidases, elle reste active dans la cellule plus longtemps et conserve une affinité optimale pour son site récepteur.

L'amidation a été largement décrite («Peptide amidation", Alan F. Bradbury et Derek G. Smyth, TIBS 16: 112-115, mars 1991 et «Functional and structural characterization of peptidylamidoglycolate lyase, the enzyme catalysing the second step in peptide amidation», A. G. Katopodis, D. S. Ping, C. E. Smith et S. W. May, Biochemistry, 30(25): 6189-6194, juin 1991), et son mécanisme est comme suit :
1 - Clivage de la chaîne du précurseur polypeptidique de l'hormone par une endoprotéase au niveau d'une séquence de deux acides aminés basiques (c'est-à-dire une séquence de deux acides aminés choisis parmi l'arginine et/ou la lysine),
2 - Par la suite, deux clivages par la carboxypeptidase, ce qui conduit à l'intermédiaire de glycine étendu,
3 - L'enzyme PAM (Peptidyl-glycine-α-Amidating Monooxygenase) comprend deux activités enzymatiques distinctes: tout d'abord, elle convertit l'intermédiaire de glycine étendu en un dérivé d' α-hydroxyglycine, la sous-unité de l'enzyme PAM impliquée étant le PHM (Peptidyl-glycine-α-Hydroxylating Monooxygenase). Le dérivé obtenu sert de substrat pour la deuxième sous-unité PAM (appelée PAL : Peptydil-α-hydroxyglycine α-Amidating Lyase), qui fixe la fonction amine de la glycine sur l'acide aminé immédiatement adjacent au côté N-terminal et libère du glyoxylate.

Cette réaction met en jeu la présence d'un site de reconnaissance sur le précurseur de l'hormone ou des hormones, un site qui comprend toujours la séquence: glycine et deux acides aminés basiques (arginine ou lysine). On sait que les hormones polypeptidiques amidées qui sont sécrétées à l'extérieur du réticulum endoplasmique comprennent une séquence consensus signal d'environ quinze à trente acides aminés, cette séquence étant présente à l'extrémité N-terminale de la chaîne polypeptidique. Elle est coupée plus tard par une enzyme signal peptidase de telle sorte qu'elle ne peut plus être trouvée dans la protéine, une fois secrétée.

Etant donné l'importance des polypeptides amidés connus dans le contexte de nombreux systèmes biologiques, on a recherché des méthodes d'identification de polypeptides amidées supplémentaires. Malheureusement, à l'heure actuelle, la découverte d'une nouvelle protéine n'est pas facile.

Jusqu'ici, il a été développé un certain nombre d'approches visant à identifier de nouvelles protéines d'intérêt biologique potentiel.

Dans l'une de ces approches, on isole de nouvelles protéines d'intérêt à partir d'une source en sélectionnant une propriété spécifique dont le chercheur pense qu'elle peut être possédée par une ou plusieurs protéines potentielles d'intérêt dans un échantillon. Conformément à cette approche, on peut isoler et purifier les protéines par des techniques variées : précipitation au point isoélectrique, extraction sélective par certains solvants puis purification par cristallisation, distribution à contre-courant, adsorption, partition ou chromatographie par échanges d'ions, électrophorèse.

Les techniques classiques d'isolement de protéines décrites ci-dessus n'offrent qu'un succès limité dans l'isolement et l'identification de nouvelles molécules biologiques d'intérêt. Cette approche implique la connaissance des propriétés de la protéine à isoler. Typiquement, lorsque l'on procède à l'isolement de protéines par utilisation d'une propriété commune, l'on fera face à l'une des deux situations ci-après. Dans la première situation, la propriété commune sera, pour la plupart du temps, non associée ou seulement associée de façon marginale à la fonction biologique des molécules en passe d'être isolées. L'on pourrait envisager, par exemple, deux protéines partageant des points isoélectriques identiques mais possédant des fonctions biologiques sans aucune relation Dans la deuxième situation, la séparation pourrait être réalisée sur la base d'une propriété commune qui est très étroitement associée à la fonction biologique de la molécule sur le point d'être isolée. Dans cette catégorie, par exemple, on pourrait envisager des molécules qui se lient à la même molécule réceptrice.Dans la première situation, l'isolement de polypeptides potentiellement nouveaux est très peu focalisée, étant donné la probabilité d'isolement de composés à fonction biologique complètement non apparentée. Tout à l'opposé, la dernière situation souffre de la carence exactement contraire en ce qu'elle ne permet l'isolement que d'un nombre très limité de molécules nouvelles intéressantes du point de vue biologique.

C'est ainsi que l'homme du métier, cherchant à isoler des polypeptides d'intérêt potentiellement nouvelles, par l'intermédiaire de techniques de séparation de protéines classiques, a été confronté au dilemme consistant à obtenir un embrouillamini de polypeptides biologiquement non apparentés ou, en solution de remplacement, de n'obtenir qu'un ensemble très spécifique de polypeptides.

Une autre carence sérieuse des techniques classiques d'isolement de nouveaux polypeptides d'un échantillon est liée à la nature de l'échantillon lui-même. Bien évidemment, il n'y aura qu'un nombre limité de polypeptides disponibles en vue de l'isolement et de l'identification dans un échantillon biologique donné. De plus, il faut prendre grand soin, dans le cas d'échantillons de ce genre, d'assurer l'intégrité continue des molécules biologiquement actives qui s'y trouvent.

Les tentatives antérieures d'isolement et de caractérisation de peptides nouveaux comprenant une extrémité C-terminale amidée ont suivi, ce qui n'est pas surprenant, l'approche classique consistant à partir d'un échantillon biologique et à-sélectionner une méthode en provenance de l'arsenal des techniques connues de séparation pour l'isolement et l'identification des peptides. Par exemple, dans le brevet américain No. 5,360,727, au nom de Matsuo et al., a été isolée une enzyme d'origine porcine à alpha-amidation sur un C-terminal, par extraction et purification de l'enzyme à partir de cordons d'oreillettes porcines manifestant l'activité enzymatique. Dans le brevet américain No. 5,871,995, émis au nom de Iida et al., on a purifié des enzymes participant à l'amidation en C-terminal à partir d'un matériau biologique comme le sérum chevalin par chromatographie d'affinité, en utilisant un produit d'addition peptide glycine à C-terminal, en tant que ligand. Dans le brevet américain No. 4,708,934, au nom de Gilligan et al., on a extrait une enzyme monooxygénase peptidyl-glycine alpha-amidante des lignées cellulaires et d'échantillons tissulaires du carcinome de la thyroïde médullaire. Là où l'identification d'un nombre substantiel de nouveaux polypeptides capables d'amidation constitue l'objectif, des techniques d'isolement classiques comme celles-ci sont complètement inappropriées, du fait qu'elles permettent typiquement l'isolement seulement d'un polypeptide unique d'intérêt à partir d'une source susceptible de contenir ce polypeptide.

Dans la demande PCT WO 99/10361, la cessionnaire de la présente demande a récemment développé une méthode qui surmonte un grand nombre des inconvénients discutés ci-dessus, dans la mesure où elle permet l'identification rapide d'une grand nombre de peptides putatifs qui comprennent une extrémité C-terminale amidée. En particulier, par opposition à des techniques antérieures qui se fondent sur une propriété physique particulière du polypeptide pour l'isoler d'une source dont on soupçonne ou dont on sait qu'elle le contient, la méthode développée par la cessionnaire se base sur une caractéristique de la séquence peptidique du précurseur de toutes les hormones amidées connues jusqu'à présent, en permettant ainsi la détection simultanée de plusieurs nouvelles hormones de cette catégorie. Plus particulièrement, cette technique se base sur l'identification directe de la séquence nucléotidique qui code pour les précurseurs dans des banques ADNc préparées à partir de tissus dans lesquels les précurseurs de ces hormones peuvent être synthétisés.

La méthode de la demande PCT WO 99/10361 permet l'identification du précurseur d'un peptide ayant une extrémité C-terminale amidée par les étapes successives suivantes :
1 - Obtention d'une banque d'ADN;
2 - Hybridation d'un ou de plusieurs oligonucléotides OX avec la banque d'ADN ;
3 - Identification de la séquence d'ADN ou des séquences d'ADN de la banque qui s'hybrident avec un oligonucléotide OX ;
4 - Identification dans cette séquence ou dans ces séquences d'un ou de plusieurs peptides ayant une extrémité C-terminale amidée possible.

OX est un oligonucléotide à brin unique qui peut s'hybrider dans des conditions douces avec un oligonucléotide OY de la séquence Y1-Y2-Y3-Y4-Y5, dans laquelle Y1 représente une séquence nucléotidique de 1 à 12 nucléotides ou Y1 est supprimé, Y2 représente un trinucléotide qui code pour Gly, Y3 et Y4 représentent indépendamment un trinucléotide qui code pour Arg ou Lys et Y5 représente une séquence nucléotidique de 1 à 21 nucléotides ou Y5 est supprimé.

La banque d'ADN est de préférence une banque d'ADNc. Une banque d'ADNc contient l'ADNc correspondant à l'ARNm cytoplasmique extrait d'une cellule donnée. La banque est dite complète si elle comprend au moins un clone bactérien pour chaque ARNm de départ. L'hybridation a lieu si deux oligonucléotides ont des séquences nucléotidiques substantiellement complémentaires, et s'ils peuvent se combiner sur toute leur longueur en établissant des liaisons hydrogène entre les bases complémentaires.

La recherche à l'aide de cette méthode de la demande PCT WO 99/10361 s'est avérée être bien moins restrictive que les techniques classiques de biochimie susmentionnées, puisque :
- elle peut conduire à l'isolement de plusieurs précurseurs distincts présents dans le-même tissu par le même principe;
- elle permet la détection, dans les mêmes conditions techniques, de précurseurs correspondant aux hormones qui ont des propriétés biochimiques et biologiques très différentes;
- elle permet l'identification concomitante de toutes les hormones peptidiques qui peuvent être contenues dans le même précurseur.

En conséquence, la technique de sélection formulée dans la demande PCT WO 99/10361 permet une économie non négligeable en temps et en argent dans un secteur où les coûts de recherche et de développement représentent une proportion très élevée du chiffre d'affaires.

En permettant l'obtention d'un grand nombre de polypeptides potentiellement utiles du point de vue thérapeutique, la technique développée permet l'étude pharmacologique des substances actives ayant un rôle physiologique fondamental dans l'organisme mammifère : hormones et plus particulièrement, neurohormones polypeptidiques amidées. Vu la disponibilité pour la première fois d'ADNc correspondant aux substances actives, il est maintenant possible d'introduire un vecteur cloné par génie génétique pour conduire à la synthèse d'hormones ayant une utilisation thérapeutique par le moyen de microorganismes.

Bien que donnant naissance à de nombreux avantages significatifs en termes de capacité d'obtention rapide d'un grand nombre de molécules peptidiques candidates putatives qui servent en tant que précurseurs aux peptides comprenant une extrémité C-terminale amidée, il reste encore certaines difficultés associées à l'utilisation d'une banque d'ADNc en vue de l'exécution de la sélection par criblage. Comme discuté antérieurement, la banque d'ADNc dérive de manière typique d'une cellule unique et ne contiendra, par conséquent, que les polypeptides qui sont exprimés dans cette cellule. Ceci veut dire que, même s'il est présent au sein du génome de la cellule, la méthode de sélection ne détectera pas un peptide putatif si ce peptide n'est pas exprimé dans cette cellule. De plus, même dans la mesure où un polypeptide d'intérêt est exprimé dans la cellule, la technique de sélection est nécessairement limitée aux polypeptides exprimés par cette cellule particulière et, de fait, par l'espèce particulière de vie de laquelle la cellule est dérivée. Ceci rend particulièrement difficile la sélection du nombre considérable de peptides putatifs qui sont d'intérêt.

La méthode de la demande PCT WO 99/10361 a ainsi résolu une restriction très importante dans l'identification de peptides putatifs servant de précurseurs de peptides comprenant une extrémité C-terminale amidée. Plus spécifiquement, alors que la méthode de la demande PCT WO 99/10361 fournit certainement les moyens grâce auxquels on peut sonder une banque d'ADNc à la recherche de toutes les séquences possibles ayant la propriété désirée d'amidation post-traduction, son application était néanmoins restreinte aux ADNc trouvés présents dans la banque d'ADNc.

Récemment, on s'est intéressé à l'utilisation des banques de données disponibles contenant des nombres considérables de séquences nucléotidiques en vue de comparer, par exemple, une séquence d'intérêt à des séquences connues.

Par exemple, dans le brevet américain No. 5,706,498, on divulgue un système d'extraction d'une banque de données en vue d'obtenir une séquence de gène ayant une séquence similaire à une donnée de séquence en provenance d'une banque de données de gènes. La banque de données de gènes stocke les données de séquence de gènes dont les structures ou séquences ont déjà été analysées et identifiées. Le système comprend une unité opérationnelle de programmation dynamique pour déterminer le degré de similarité entre les données cible et les données clé en utilisant les données des séquences de bases du gène en provenance de la banque de données de gènes en tant que données cible et les données des séquences de bases en tant que clé pour l'extraction, et une unité de traitement centrale pour exécuter le processus d'accession aux données, afin de permettre l'accès à la banque de données de gènes, en parallèle au processus opérationnel pour déterminer le degré de similarité, en transmettant les données de séquence des bases de la banque de données de gènes en continu les unes après les autres vers l'unité opérationnelle de programmation dynamique en tant que données cible, en contrôlant la banque de données de gènes et l'unité opérationnelle de programmation dynamique.

Le brevet américain No. 5,873,082 divulgue une méthode de recherche dans une banque de données de gènes, caractérisée en ce qu'une séquence homologue d'une séquence donnée fait l'objet d'une recherche dans la banque de données de gènes et en ce que les résultats sont formulés sous forme de sorties classées en séquence d'homologie croissante. Conformément au brevet, on peut comparer de manière efficace une multiplicité de listes ayant des similarités et des différences. Dans le cas des résultats de recherche de la banque de données de gènes, on peut comparer rapidement un grand nombre de listes, y compris un nombre énorme de noms de séquences.

Le brevet américain No. 5,577,249 divulgue une méthode de localisation d'une séquence de référence dans une banque de données. Le mode de réalisation le plus préféré a une application spécifique à la recherche du génome d'organismes vivants, en particulier du génome humain, pour trouver les emplacements et les utilités des séquences nucléotidiques et d'autres informations biologiques que l'on trouve sur des séquences d'ADN. La méthode emploie des banques de données du génome humain disponibles commercialement, qui possèdent des sous-séquences des chaînes d'ADN décomposés en séquences nucléotidiques *token.* Un index original unique associé au brin d'ADN original est alors créé. Une séquence nucléotidique de référence est sélectionnée. Les index de référence et les index d'origine sont comparés. La méthode a été appliquée à l'appariement des séquences de référence de nucléotides pour le génome de *E. coli* qui contient approximativement 4 millions de nucléotides.

Le brevet américain No. 5,701,256 divulgue une méthode et un appareillage destinés à des comparaisons de séquences, caractérisés en ce que l'on compare de nouvelles séquences de protéines à des séquences connues, en provenance par exemple d'une banque de données de séquences, de manière typique afin de déterminer quel niveau de similarité est commun aux protéines en termes de caractéristiques structurales et fonctionnelles.

Le brevet américain No. 5,523,208 divulgue une méthode de criblage de banques de données de nucléotides ou de séquences d'ADN pour identifier les régions génétiques ou les gènes codant pour des protéines entrant en interaction du point de vue biologique. En particulier, la méthode fournit un moyen de criblage de banques de données se composant de matériau génétique cloné, y compris mais sans être limité à l'ADN, à l'ARN, à l'ARNm, à l'ARNt et aux fragments nucléotidiques, pour identifier la fonction génétique du matériau génétique de fonction inconnue. La méthode, en cas d'utilisation sur des fragments d'ADN de potentiel de codage inconnu, produira une liste de fragments de gènes qui codent pour des protéines ayant le potentiel de formation de complexes ou de configurations multimères avec la protéine inconnue.

Le document Nevill-Manning C.G. et al. : "Highly specific protein sequence motifs for genome analysis." Proc. Natl. Acad. Sci. USA, vol. 95, 1998, pages 5865-5871, décrit une méthode pour assigner une fonction à des gènes nouvellement séquencés, par criblage de banques de données à la recherche d'un alignement de séquence donné. Ce criblage de banques de données est réalisé à partir d'un motif de séquence protéique obtenu après traduction de nouveaux gènes identifiés et comprend en outre la vérification de la présence de cadre de lecture ouvert (ORF).

Comme la discussion ci-dessus le démontre, une des applications majeures des méthodes informatisées de recherche de banques de données d'informations génétiques est la comparaison d'une séquence nouvellement trouvée de fonction inconnue à une banque de données de séquences de fonction connue. L'objectif de méthodes de ce genre est, bien entendu, d'établir la fonction de la protéine inconnue en la rapprochant de protéines similaires du point de vue de la structure, à fonction connue. Une autre application des méthodes informatisées est de trouver les séquences dans une banque de données qui sont liées du point de vue de la structure. Encore d'autres méthodes tendent de trouver des séquences qui forment des complexes avec une séquence connue. Le point central de toutes ces méthodes est en général la comparaison d'une séquence à une autre séquence dans l'optique de déterminer lesquelles des séquences sont similaires du point de vue de la structure, plutôt que de déterminer quels gènes, en provenance d'une grande banque de données de gènes, possèdent une propriété biologique donnée, même si des gènes de ce genre ne sont pas strictement similaires du point de vue de la structure.

### RESUME ET OBJETS DE L'INVENTION:

La présente invention a pour objet primaire de surmonter les inconvénients relatifs aux techniques de l'art antérieur visant à l'identification de molécules intéressantes du point de vue biologique choisies parmi un grand groupe de molécules candidates. Cet objet est atteint en fournissant une méthode selon la revendication 1 d'identification des peptides putatifs d'une fonction donnée choisis parmi des séquences nucléotidiques ou des séquences peptidiques de fonction inconnue par criblage d'une banque de données à la recherche de la présence d'une combinaison particulière de nucléotides ou d'acides aminés indicative des peptides de fonction donnée.

La présente invention met à disposition une méthode d'identification d'un peptide putatif d'une fonction donnée qui n'est pas limitée à ces peptides exprimés dans une source biologique particulière, par exemple parce que la protéine n'est pas exprimée dans cette source.

La présente invention met à disposition une méthode d'identification d'un peptide putatif d'une fonction donnée qui ne dépend pas, pour l'identification, des propriétés physiques du peptide, comme le point isoélectrique ou la solubilité.

La présente invention met à disposition une méthode d'identification d'un peptide putatif d'une fonction donnée qui est applicable aux protéines qui sont par ailleurs non apparentées dans leurs propriétés physiques.

La présente invention met à disposition une méthode d'identification d'un peptide putatif d'une fonction donnée choisi parmi des polypeptides candidats qui manifestent un très faible degré d'homologie les uns avec les autres.

La présente invention met à disposition une méthode d'identification d'un peptide putatif d'une fonction donnée qui facilite l'étude pharmacologique de substances actives ayant une rôle physiologique fondamental dans un organisme comme les hormones, et, plus particulièrement, les hormones polypeptidiques amidées.

La présente invention met à disposition une méthode d'identification d'un peptide putatif d'une fonction donnée qui peut être exécutée à l'aide des banques de données génétiques disponibles et des logiciels disponibles.

En résumé, la présente invention met à disposition une méthode d'identification d'un précurseur d'un peptide comprenant une extrémité C-terminale amidée, comprenant les étapes suivantes :
(i) obtention d'une banque de données de polynucléotides ou de polypeptides ;
(ii) criblage de la banque de données à la recherche de la présence d'une combinaison de nucléotides ou d'acides aminés indicative du précurseur du peptide comprenant l'extrémité C-terminale amidée ;
(iii) identification des séquences polynucléotidiques ou polypeptidiques, qui comprennent la combinaison de nucléotides ou d'acides aminés indicative du précurseur du peptide comprenant l'extrémité C-terminale amidée.

La banque de données comprend, de préférence, des séquences polynucléotidiques et/ou des séquences polypeptidiques correspondant aux séquences polynucléotidiques, et, en option, des numéros d'accession pour les séquences polynucléotidiques. Là où les séquences polypeptidiques ne sont pas disponibles dans la banque de données, elles peuvent être obtenues par traduction des séquences polynucléotidiques dans ladite banque de données. Dans un mode de réalisation préféré, trois séquences polypeptidiques différentes sont obtenues, correspondant à la traduction de trois cadres de lecture différents desdites séquences polynucléotidiques.

La banque de données peut, en outre, comprendre une information d'annotations se rapportant aux séquences polypeptidiques ou aux séquences polynucléotidiques, comme au moins une information choisie parmi l'origine, la source, les caractéristiques et les références pour les séquences.

Lors du criblage de la banque de données, on préfère d'abord localiser le codon d'initiation AUG dans une séquence polynucléotidique et vérifier le fait qu'aucun cordon d'arrêt n'est présent entre ledit codon d'initiation AUG et la combinaison de nucléotides indicative du précurseur du peptide comprenant l'extrémité C-terminale amidée.

Une fois effectuée l'étape d'identification des séquences nucléotidiques ou polypeptidiques, les séquences identifiées peuvent être comparées à des séquences de fonction biologique connue et aux séquences identifiées dont la fonction biologique est inconnue.

Alternativement, une fois effectuée l'étape d'identification des séquences nucléotidiques ou des séquences polypeptidiques, la similarité des séquences sélectionnées d'activité biologique inconnue peut être comparée aux séquences de fonction connue et, si aucune séquence similaire n'est trouvée, la séquence d'activité biologique inconnue peut être sélectionnée en vue d'un examen supplémentaire. Si une séquence similaire est trouvée, la séquence d'activité biologique inconnue peut être sélectionnée en tant que séquence candidate manifestant la fonction putative de la séquence similaire connue.

Dans un mode de réalisation, la séquence polypeptidique identifiée peut être obtenue et les propriétés de la séquence polypeptidique peuvent être évaluées.

La combinaison des nucléotides comprend la séquence Y1-Y2-Y3-Y4-Y5, dans laquelle Y1 est une séquence nucléotidique de 1 à 12 nucléotides ou est supprimée, Y2 est un codon pour Gly, Y3 et Y4 indépendamment sont des codons pour Arg ou Lys et Y5 est une séquence nucléotidique de 1 à 21 nucléotides ou Y5 est supprimé.

Par référence à ce qui précède ainsi qu'à d'autres objets, caractéristiques et avantages de l'invention qui deviendront apparents ci-après, l'invention peut être mieux comprise en se rapportant à la description détaillée des modes de réalisation-préférés et de la revendication adjointe.

### DESCRIPTION DETAILLÉE DES MODES DE RÉALISATION PRÉFÉRÉS:

Par «peptides putatifs d'une fonction donnée», on entend des polypeptides provenant d'une séquence oligonucléotidique particulière qui est caractéristique d'une fonction particulière partagée par un grand nombre de protéines parmi une espèce unique et/ou parmi des espèces différentes. En particulier, certaines séquences oligonucléotidiques se sont avérées être associées à certains types de protéines, comme des hormones à amidation en C-terminal ou des amylases. L'invention est applicable à chaque fois qu'il existe une séquence oligonucléotidique indicative d'une fonction de ce genre.

Par «précurseur d'un peptide comprenant une extrémité C-terminale amidée », on entend n'importe quelle protéine précurseur qui subit une réaction d'amidation à l'extrémité C-terminale, comme décrit, par exemple chez Bradbury et al.

Par «banque de données de polynucléotides ou de polypeptides», on entend l'une quelconque des banques de données disponibles publiquement, comme FASTA, GENBANK, EMBL et SP-TREMBL, PIR, REBASE, PROSITE ou SWISS-PROT, qui comprennent, de manière typique, des données de séquences polynucléotidiques et/ou de séquences polypeptidiques. Les données de séquences nucléotidiques sont disponibles, par exemple, auprès des organismes EMBL, GENBANK et à d'autres endroits comme EXPASY. Les données de ce genre comprendront également souvent des numéros d'ACCESSION.

Si les séquences peptidiques correspondant à un numéro d'ACCESSION donné (par exemple dans SWISS-PROT), cette séquence «validée» est incluse dans la banque de données. Dans le cas contraire, la séquence nucléotidique est de préférence traduite par utilisation de programmes disponibles dans l'art comme Translate. La traduction est effectuée en utilisant les données associées à la séquence nucléotidique (extrémité 3' ou 5') et trois cadres de lecture différents (N, N+1, N+2). Si cette information n'est pas disponible, la traduction est effectuée en utilisant tout à la fois la séquence nucléotidique disponible et sa séquence complémentaire (six peptides putatifs pour une séquence nucléotidique unique).

En option, des banques de données de ce genre comprennent en outre des annotations contenant toutes les informations qui sont disponibles pour une séquence particulière, comme ORIGIN [ORIGINE], SOURCE [SOURCE], FEATURES [CARACTÉRISTIQUES], REFERENCES [RÉFÉRENCES] associées et COMMENTS [COMMENTAIRES] associés à la séquence. Ceci facilite une extraction complémentaire de la banque de données. Un exemple de l'information disponible pour annotation est donné ci-après:
- LOCUS: HUMXT00347 239 bp mRNA EST 24-JUN-1992
- DEFINITION: Human expressed sequence tag (EST00347 similar to Repeat: CT), mRNA sequence.

### ACCESSION M62275

- NID: g340398
- KEYWORDS EST;: expressed sequence tag.
- SOURCE: Homo sapiens (library: Stratagene catalog #936205) female 2 yr old
Hippocampus cDNA to mRNA.
- ORGANISM: Homo sapiens
Eukaryotae; mitochondrial eukaryotes; Metazoa; Chordata; Vertebrata; Eutheria; Primates; Catarrhini; Hominidae; Homo.
- REFERENCE: 1 (bases 1 to 239)
- AUTHORS: Adams,M.D., Kelley,J.M., Gocayne,J.D., Dubnick,M., Polymeropoulos,M.H., Xiao,H., Wu,A., Olde,B., Moreno,R.F., Kerlavage,A.R., McCombie,W.R. and Venter,J.C. JOURNAL Science 252, 1651-1656 (1991)
- TITLE: Complementary DNA sequencing: Expressed sequence tags and human genome project
- MEDLINE: 91262645
- FEATURES: Location/Qualifiers
source 1..239
/organism="Homo sapiens"
/db_xref="taxon:9606"
/dev_stage="2 yr old"
/sex="female"
/tissue_type="Hippocampus"
/tissue_lib="Stratagene catalog #936205"

| | | | | | |
|---|---|---|---|---|---|
| BASE COUNT | 36 a | 72 c | 33 g | 97 t | 1 others |
| ORIGIN | | | | | |

1 ttcatgctca tgtaaccttc ttaatagtgc cttgtctgct gggtttgtag ctgtaagagt
61 tctgcaaact ggccctataa aaatattgat gctgtccatt aaaatgaatc tctctctctc
121 actcagtctc tctctctgtc tgtctctctt tcttctctct cctgccatgt gtgtgtctct
181 ctctactcct ctgattttgn cctctctctc tattctgcta ctctctctcc tctcctccg

Par «combinaison de nucléotides ou d'acides aminés indicative du précurseur du peptide comprenant l'extrémité C-terminale amidée», on veut dire, de préférence, la séquence Y1-Y2-Y3-Y4-Y5, dans laquelle Y1 est une séquence nucléotidique de 1 à 12 nucléotides ou est supprimée, Y2 est un codon pour Gly, Y3 et Y4 indépendamment sont des codons pour Arg ou Lys et Y5 est une séquence nucléotidique de 1 à 21 nucléotides ou Y5 est supprimé. La combinaison particulière est formulée en détail dans la demande PCT WO 99/10361, la divulgation duquel est incorporée ici à titre de référence.

Dans le mode de réalisation préféré, on obtient la banque de données à cribler en faisant appel à un script au script SQL/plus similaire au script qui suit :

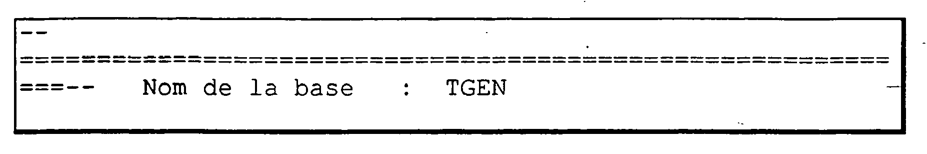

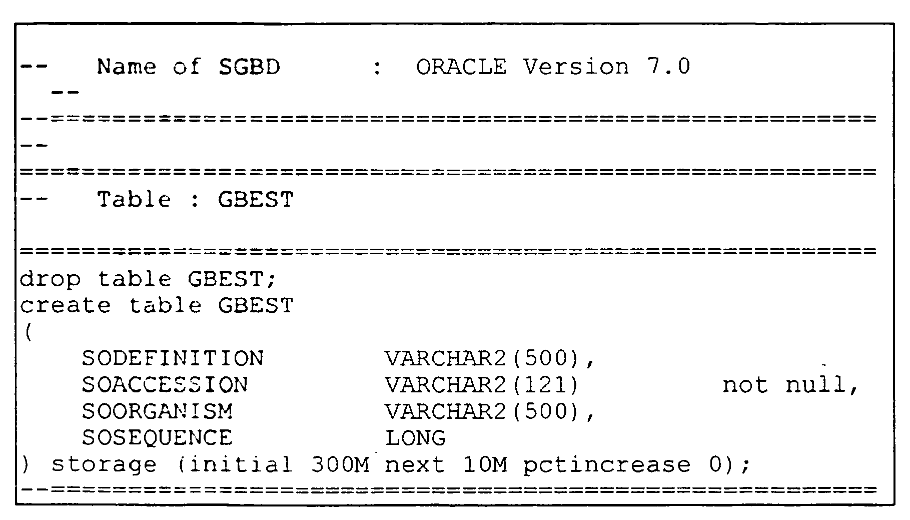

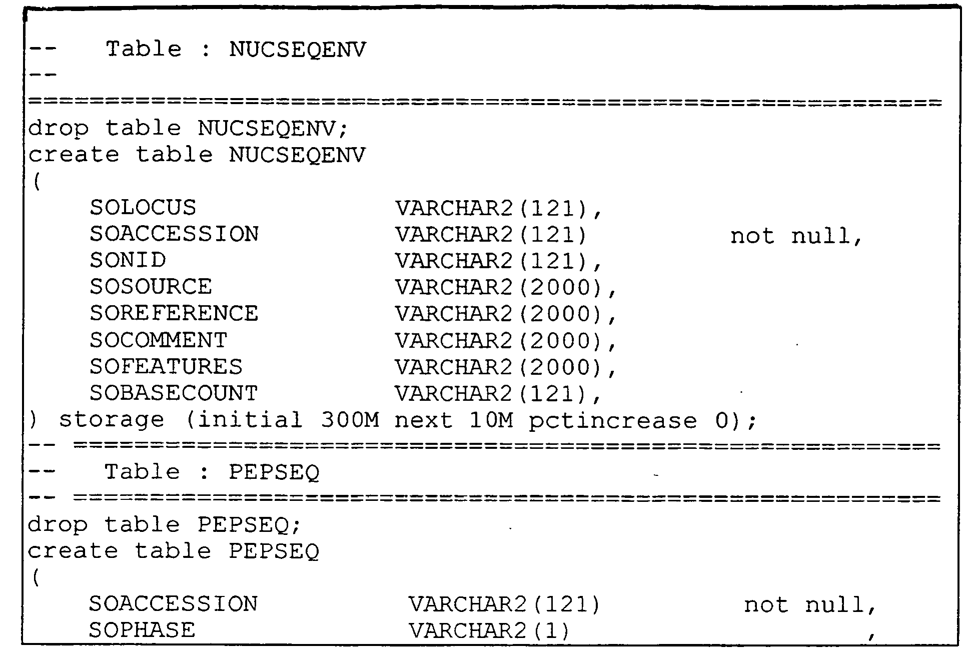

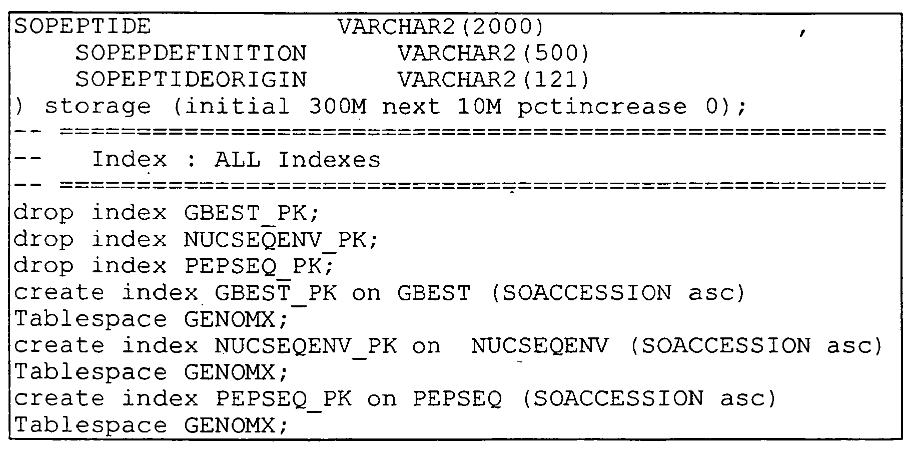

Il est bien évident que le nombre et la taille des différents champs d'ORACLE peut être ajustée de manière à s'adapter au nombre et à la taille des données qui sont importées dans les tables. Si un autre type d'information devait être ajouté aux bases de données publiques, un nouveau champ serait créé de façon correspondante dans ORACLE.

Par exemple, les champs SOLOCUS, SODEFINITION, SOACCESSSION, SOORGANISM, SONID, SOKEYWORDS SOSOURCE, SOREFERENCE, SOCOMMENT, SOFEATURES, SOBASECOUNT , SOSEQUENCE dans oracle contiennent respectivement les saisies LOCUS, DEFINITION, ACCESSSION, ORGANISM.

NID, KEYWORDS, SOURCE, REFERENCE, COMMENT, FEATURES, BASECOUNT , SEQUENCE des fichiers de données de la banque génétique.

SOPHASE contient le cadre de lecture (0, +1, +2) si la séquence peptidique est engendrée par utilisation du traducteur d'ADN IHB, SOPEPTIDE contient la séquence peptidique correspondant à un SOACCESSION donné, finalement SOPEPTIDEORIGIN indique comment la séquence peptidique a été obtenue (IHB's ADN Translator, GENBANK, SWISS-PROT, etc.).

Si un fichier formaté FASTA (voir ci-dessous) est utilisé à la place d'un fichier formaté GENBANK, la ligne d'en tête est stockée dans le champ SODEFINITION quand on a affaire à une séquence nucléotidique et quand la séquence nucléotidique elle-même est stockée dans le champ SOSEQUENCE. Si la donnée en passe d'insertion dans la banque de données est une séquence peptidique, la ligne d'en-tête est stockée dans SOPEPDEFINITION et la séquence peptidique est stockée dans le champ SOPEPTIDE, une fois de plus SOPEPTIDEORIGIN indiquera d'où provient la séquence peptidique (GENBANK, SWISS-PROT, etc.)
.gi1402336 (M17352) dnaN protein [Salmonella typhimurium]
MKFTVEREHLLKPLQQVSGPLGGRPTLPILGNLLLQVADGALSLTGTDLEMEMVARVTLSQP
>gi|306148 (L19604) core polypeptide [Heliobacillus mobilis] 
MATADAAFNPRAQVFEWFKDKVPATRGAVLKAHINHLGMVAGFVSFVLVHHLSWLSDQVLFAPTPIFYAR

LYQLGLDASARSADALMVARLHLPAAIIFWIIGHIKTPREDEFLKNVTFGKTLVAQFHFLALVATLWGMH

MAYIGVRGANGGIVPTGLSFDMFGPITGATLAGNHVAFGALLFLGGVFHHFAGFNTKRFAFFEKDWEAVL
 
SVSAQVLAFHFATVVFAMIIWNRPDQPILSFYFMQDYALSNYAAPEIREIASQNPGFLIKQVILGHLVFG

VMFWIGGVFHGASLHVRATNDPKLAEALKDFKMLKRCYDHDFQKKFLALIMFGAFLPIFVSYGIATHNTI

SDLHHLAKAGMFANMTYINIGTPLHDAIFGSHGTVSDFVAAHAIAGGLHFTMVPLWRMVFFSKVSPWTTK

VGMKAKRDGEFPCLGPAYGGTCSISLVDQFYLAIFFSLQVIAPAWFYLDGCWMGSFVATSSEVYKQAAEL

FKANPTWFSLHAVSNFTSEVTSATSSLKPLVCSNTTMVTWFKPCWAAHFIWAFTFSMLFQYRGSRDEGAM

VLKWAHEQVGLGFAGKVYNRALSLKEGKAIGTFLFFKMTVLCMWCLAMV
 >gil 153320 (L05390) hydroxylase [Streptomyces haistedii]

MNARADRAGDTVHRVPVLVVGGSLVGLSTSVFLGRLGVRHMLVERHAGTSVHPRGRGNNVRTMEVYRAAG

VEQGIX.

Lors de la compilation de la banque de données en vue du criblage, on procède à la lecture et à la manipulation des formats de fichiers standard utilisés pour sauvegarder les programmes «High Throughput Sequencing» [séquençage à débit élevé] et d'autres programmes «Genome» (par exemple FASTA, GENBANK). Les portions de données qui sont pertinentes par rapport à l'étape de criblage, par exemple les données d'annotation, sont alors identifiées. Les champs résultants sont insérés dans Oracle. Si la séquence nucléotidique est une extrémité 5', la séquence est directement traduite en une séquence peptidique, s'il s'agit d'une extrémité 3', la séquence complémentaire de la séquence donnée est engendrée et utilisée pour la traduction (la phase de traduction prend en compte les trois cadres de lecture possibles pour engendrer la séquence peptidique). La dernière étape met en jeu la prédiction de la structure secondaire du peptide, qui peut être basée sur la théorie de l'information tel que le programme qui a été développé par J.Garnier, D.Osguthorpe et B. Robson. Le logiciel utilise toutes les fréquences d'appariement au sein d'une fenêtre de 17 résidus d'acides aminés. Après une contre validation sur une banque de données de 267 protéines, la prédiction a une précision moyenne de 64,4% pour une prédiction à trois états (hélice, brin bêta et enroulé). Le programme produit deux sorties de données, l'une donnant la séquence et la structure secondaire prédite, l'autre donnant les valeurs des probabilités pour chaque structure secondaire à chaque position d'acide aminé. La structure secondaire prédite est celle de la probabilité la plus élevée compatible à un segment d'hélice d'au moins quatre résidus et à un segment étendu (brin bêta) d'au moins deux résidus.

Une fois rassemblées les données pertinentes en provenance de la banque de données, on peut cribler une banque de données de ce genre à la recherche de la présence d'une combinaison de nucléotides ou d'acides aminés indicative du précurseur du peptide, de préférence de ceux comprenant l'extrémité C-terminale amidée. Le but de cette étape est, bien entendu, de convertir la quantité énorme de données non triées en un ensemble limité de peptides putatifs d'intérêt pharmaceutique (hormones ou fragments d'hormones potentiels ou ligands récepteurs endogènes et produits similaires). La description générale du processus est suivie d'une application à une recherche de peptides amidées potentielles dans la banque de données Expressed Sequence Tags.

Le processus général peut notamment se dérouler comme suit :
- Extraction de séquences automatisées d'une source publique, comme l'Internet (EMBL, GENBANK, SWISS-PROT, PDB, etc.)
- Analyse des fichiers et importation des données dans ORACLE
- Sélection des séquences à partir d'un sous-ensemble (par exemple GENBANK EST) ou à partir de la banque de données toute entière
- Recherche de toutes les séquences manifestant un motif spécifique d'intérêt, comme les précurseurs d'un peptide comprenant une extrémité C-terminale amidée
- Vérification de l'absence de tout codon d'ARRET entre le codon AUG indiquant le début du cadre de lecture et le motif recherché
- Sélection des séquences de fonction biologique inconnue
- Vérification de ce que, si on le trouve, le motif est un cadre de lecture ouvert (séquence consensus Kozak)
- Comparaison de l'environnement de l'emplacement du motif à celui requis (par exemple structures secondaires requises autour d'un site de maturation telle que la proximité d'hélices alpha ou de feuilles bêta)
- Vérification de la présence d'une similarité des séquences et d'autres séquences connues (champ DÉFINITION)
- Utilisation de techniques de "filtration successive" ["threading" en anglais] pour rechercher des séquences manifestant une structure secondaire similaire, si aucune structure similaire n'est définie dans la banque de données
- Si on ne trouve aucune séquence similaire, sélection de la séquence en tant que candidat synthétique, dont la fonction doit être déterminée
- Si des séquences similaires de fonction connue sont trouvées, les séquences peuvent être sélectionnées en tant que candidate synthétique dont la fonction putative est celle de la séquence similaire

Les exemples suivants sont donnés à titre d'illustration et ne devraient pas être interprétés comme limitant la matière traitée divulguée et revendiquée.

### Exemples

En utilisant la méthode décrite ci-dessus, on a identifié les peptides suivants :

| **Numéro** | **Séquence** | **Origine** |
|---|---|---|
| 1 | H-GQDSIEPVPGQK-NH2 | DbEST |
| 2 | H-YARVQVVA-NH2 | pré-pro-bradykinine |
| 3 | H-YFKIDNVKKARVQWA-NH2 | pré-pro-bradykinine |
| 4 | H-PLEPSGG-NH2 | Gène HYAA |
| 5 | H-ELGRGPGPPLPERGA-NH2 | Gène HYA22 |
| 6 | H-YERNRQAAAANPENSRGK-NH2 | Facteur neurotrophique dérivé de la ligne de cellule de gliale |
| 7 | H-SLLSKVSQ-NH2 | Protéine réparant l'ADN (cellules XP-C) |
| 8 | H-VTQDPKLQM-NH2 | Précurseur de glycoprotéine CD4 (surface cellules T) |
| 9 | H-DFPEEVAIVEEL-NH2 | Précurseur du glucagon |
| 10 | H-MDVGGLSDPYVKVHLLQG-NH2 | Synaptotagmine V |
| 11 | H-DPSLPVASSSSSSSKR-NH2 | Protéine réparant l'ADN et complémentant les cellules XP-C |
| 12 | H-PLLGSTLFIPI-NH2 | Précurseur de D-β-hydroxybutyratedéhydrogénase |
| 13 | H-DSGFQMNQLR-NH2 | Précurseur de sérotransferrine (sidérophiline) |
| 14 | H-LQLEETMPSPY-NH2 | β isozyme d'ADN topoisomérase II |
| 15 | H-FSIATLRDFGV-NH2 | |
| 16 | H-FSVTTMRDFGM-NH2 | Cytochrome P450 (inductible par le phénobarbital) |
| 17 | H-DSSHAFTLDELR-NH2 | Précurseur de mélanotransferrine |
| 18 | H-DMVVFLDGGQLGTLV-NH2 | |
| 19 | H-LHISHDMTGPD-NH2 | |
| 20 | H-SLEGIFDDIVPD-NH2 | Protéine 1 induisant l'invasion de T-lymphomes et de métastases |
| 21 | H-SNYFMPFSA-NH2 | Cytochrome P450 (méphénytoïne-4-déhydroxylase) |
| 22 | H-SDAFVPFSI-NH2 | Cytochrome P450 |
| 23 | H-RTGALVLSRG-NH2 | Précurseur de leukosialine (sialoglycoprotéine des leucocytes) |
| 24 | H-ESSFQPEAGF-NH2 | |
| 25 | H-GGPISFSSSRS-NH2 | Chaîne lourde de la myosine (type B non musculaire) |
| 26 | H-IEKEAAQLQ-NH2 | |
| 27 | H-SDTSLTWNSVK-NH2 | Précurseur de lactotransferrine (lactoferrine) |
| 28 | H-FSLMTLRNFGM-NH2 | Cytochrome P450 (S-méphénytoïne 4-hydroxylase) |
| 29 | H-FQLPLDKGN-NH2 | |
| 30 | H-SFSIIGDFQN-NH2 | Précurseur du facteur de Von Willebrand |
| 31 | H-ALEKLDGTEVN-NH2 | Facteur d'épissage pré-ARNm SRP75 |
| 32 | H-VAEIQGHAG-NH2 | Précurseur de protéine morphogénétique osseuse 4 |
| 33 | H-LHNILGVETGGPG-NH2 | |
| 34 | H-SASDLTWDNLK-NH2 | Précurseur de sérotransferrine |
| 35 | H-ISELFTLE-NH2 | ADN polymérase ε (sous-unité catalytique A) |
| 36 | H-GGPGGPPGPLMEQMG-NH2 | Protéine de liaison aux ARN |
| 37 | H-ALEWLGADRNE-NH2 | Protéine associée à la FBKP-rapamycine |
| 38 | H-DVDFEGTDEPIF-NH2 | Fragment de protéine hypothétique |
| 39 | H-GATPGKALVATP-NH2 | Nucléoline |
| 40 | H-MKGPEVMAFIEQ-NH2 | Tyrosine protéine kinase |
| 41 | H-ESKLERTPQKNVQ-NH2 | Sous-unité de l'activateur 1 |
| 42 | H-PRATTPKTVRS-NH2 | Histone H1T |
| 43 | H-MKTRQNKDSMSMRS-NH2 | Protéine ribosomale S18 |
| 44 | H-ERMGHHDDYYSRLR-NH2 . | Facteur auxiliaire de la ribonucléoprotéine nucléaire courte |
| 45 | H-LVEHYPEFI-NH2 | |
| 46 | H-FSVSTLRNLGL-NH2 | |
| 47 | H-SGTLIKIFQAS-NH2 | |
| 48 | H-FAEQDAKEEANKAM-NH2 | Protéine liante FK506 (peptidyl-prolyl cis-trans déisomérase) |
| 49 | H-ETKHGGHKN-NH2 | Aspartyl/asparaginyl β-hydroxylase |
| 50 | H-LQEALSKAA-NH2 | Protéine hypothétique |
| 51 | H-PWTAVDTSVD-NH2 | Précurseur de récepteur cations-indépendant mannose-6-phosphate |
| 52 | H-VIQYFASIAAIGDR-NH2 | Chaîne lourde de la myosine (isoforme α, muscle cardiaque) |
| 53 | H-GYIGWNRSQKDID-NH2 | Dynamine-1 |
| 54 | H.QLWDVAHSVKEKF-NH2 | Glycogène synthase (foie) |
| 55 | H-GNETSFVPSRRSG-NH2 | Chaîne lourde de la myosine (isoforme des muscles lisses) |
| 56 | H-TDIFGVEETAI-NH2 | Protéine 114 associée au splicéosome |
| 57 | H-OTAVTAVEKPAPK-NH2 | |
| 58 | H-EVAMDDHKLSLDEL-NH2 | Chaîne α-2 de l'ATPase transportant le sodium et le potassium |
| 59 | H-TVTPAKAVTTP-NH2 | Nucléoline |
| 60 | H-MEAETGSSVET-NH2 | |
| 61 | H-QWAQFKIQWNQRW-NH2 | |
| 62 | H-DYSKGITVTKND-NH2 | Protéine hypothétique |
| 63 | H-VIQYLAHVASSHK-NH2 | Chaîne lourde de la myosine (type B non musculaire) |
| 64 | H-YPKPQQFFGLM-NH2 | |
| 65 | H-EPPKEETAQLTGPEA-NH2 | Grande protéine BAT-2 riche en proline |
| 66 | H-YMHGHRAPG-NH2 | |
| 67 | H-MGKWHVG-NH2 | |
| 68 | H-SSSHSLSHK-NH2 | |
| 69 | H-HVGLLRIK-NH2 | |

### Propriétés pharmacologiques des peptides identifiés :

### 1) Méthodes:

### Test de liaison: protocole alternatif par Skatron.

Les conditions expérimentales sont identiques pour tous les protocoles de test de liaison, mis à part le fait que :
i) le volume R en fonctionnement est de 200 µl ;
ii) le test est effectué dans des plaques Falcon à 96 puits ;
iii) la réaction est directement arrêtée par filtration sur un dispositif filterMat (ref. 11734) Skatron et la radioactivité associée au filtrat est évaluée:
   - soit directement à l'aide d'un compteur γ pour les peptides marqués à l'iode 124 ;
   - soit à l'aide d'un compteur γ en présence de 5 ml de liquide de scintillation.

### Préparations de membranes de cerveau de cobaye et test de liaison :

Les cobayes sont sacrifiés par rupture de leurs vertèbres cervicales, décapités et les cervelles sont retirées très rapidement et placées dans un tampon sucrose-Tris-HCl (0,32 M sucrose ; 5 mM Tris-HCl ; 0,1 g/l bacitracine) à 4 °C (environ 10 ml de tampon par cerveau). Les cervelles sont alors homogénéisées à l'aide d'un dispositif Potter et l'homogénéisat est placé en incubation pendant 30 minutes à 37 °C sous agitation, et alors centrifugé à deux reprises pendant 35 minutes à 100 000 x g à 4 °C. La teneur en protéines des préparations de membrane est évaluée conformément à la méthode de Bradford (BioRad, selon le protocole du fabricant).

### Liaison d'agonistes marqués à la préparation de membranes de cervelle de cobaye

Les membranes sont placées dans un tampon (50 mM de Tris-HCl, 5 mM de MgCl₂ et 0,1 g/l de bacitracine) à la concentration de protéines désirée (liaison de CCK iodée : 0,1 mg de protéines/ml; liaison de gastrine iodée : 0,5 mg de protéines/ml). Elles sont alors incubées en présence d'un ligand marqué dans un volume total de 500 µl (environ 10 pM pour la CCK₈ iodée, 20 pM pour la gastrine₁₃ iodée) pendant 50-80 minutes à 25 °C, et en présence ou en l'absence d'agonistes froids. La réaction est arrêtée à l'aide de 3 ml de tampon supplémenté en BSA (20 g/l) à 4 °C, les tubes sont centrifugés à 10 000 x g, le surnageant est aspiré et la radioactivité associée au précipité est évaluée à l'aide d'un compteur γ.

### Liaison d'agonistes radiomarqués à des cellules Jurkat T :

### Conditions de culture des cellules de la ligne lymphocytique humaine Jurkat T :

Les cellules Jurkat sont cultivées dans un milieu RPMI 1640 supplémenté en sérum de veau foetal (10% volume/volume) et d'antibiotiques (50 U/mL de pénicilline et 50 µg/ml de streptomycine) dans un incubateur humide à 37 °C sous une atmosphère de 5% de CO₂ dans l'air.

### Test de liaison:

Les cellules sont obtenues par centrifugation (514 g, 5 minutes), puis sont lavées à deux reprises dans un milieu standard contenant: 98 mM de NaCl ; 6 mM de KCl ; 2,5 mM de NaH₂PO₄;1,5 mM de CaCl₂ ; 1 mM de MgCl₂ ; 5 mM de pyruvate de Na ; 5 mM de fumarate de Na ; 5 mM de glutamate de Na ; 2 mM de glutamine ; 11,5 mM de glucose ; 24,5 mM de Hepes (acide N-[2-hydroxyéthyl]pipérazine-N'-[2-éthanesulfonique]) ; 0,5 g/l de bacitracine ; 0,1 g/l d'agent inhibiteur de la trypsine du soja, pH 7,4.

Les expériences des liaisons d'agonistes marquées à l'iode 125 (environ 50 picomolaires) sont effectuées à 37 °C sous agitation pendant 45-60 minutes dans un volume final de 0,5 ml de milieu standard contenant 2 x 10⁶ cellules (4 x 10⁶ cellules/ml) et en présence ou dans l'absence de compétiteurs. La liaison non spécifique est évaluée en présence d'une concentration micromolaire de peptide homologue froid.

### Préparations de membranes de différents tissus et organes du rat:

1) Les rats sont sacrifiés par rupture de leurs vertèbres cervicales, et les différents tissus sont retirés et lavés à plusieurs reprises dans un volume important de NaCl à 9 par mille.

Les étapes suivantes sont toutes effectuées à 4 °C.
2) Les tissus et les organes sont transférées, toujours séparément, dans un tampon de sucrose contenant:
   - 0,25 M de sucrose
   - 25 mM de TRIS-HCl, pH de 7,4
   - 0,2 mM de PMSF
   - 0,1 mM de 1-10 phénantroline
   - 100 µg/ml de STI

Ils sont alors soigneusement émincés à l'aide de ciseaux fins et broyés à l'ultraturax.
3) Les tissus et les organes sont finalement broyés en utilisant un appareillage Potter (10 allers-retours minimum à 1000 tours par minute).
4) Les broyats sont centrifugés à 500 g et pendant 10 minutes à 4 °C. Les surnageants sont conservés, les culots sont repris dans un tampon sucrose et centrifugés à nouveau dans les mêmes conditions.
5) Les 2 surnageants sont alors mélangés et centrifugés à 100 000 x g pendant 30 minutes à 4 °C.
6) Les surnageants sont éliminés, et les culots repris à nouveau dans un tampon sucrose pour une deuxième centrifugation à 100 000 x g pendant 30 minutes à 4 °C.
7) Les surnageants sont éliminés, et les culots repris dans un tampon *binding* contenant 50 mM de tampon TRIS-HCl (pH 7,4) et 5 mM de MgCl₂.
8) La teneur en protéines des suspensions membranaires est évaluée à l'aide d'un dosage de Bradford (BioRad, conformément au protocole du fabricant). Elles sont aliquotées et stockées dans l'azote liquide ou à -80 °C.

Les tests de liaison sont effectués conformément à un protocole identique à celui utilisé pour les membranes de cervelle de cobaye.

### Études de liaison d'agonistes marqués sur la lignée cellulaire HeLa

Les cellules sont cultivées dans un milieu DMEM additionné de sérum de veau foetal (10 %), de 0,5% d'antibiotiques (pénicilline /streptomycine) et de 0,5% de glutamine.
24 à 48 heures avant l'expérimentation, on procède à la réplication des cellules sur des plaques à 24 puits avec environ 100,000 cellules par puits et par ml.

Les études de liaison sont effectuées avec un tampon BindH

| | | |
|---|---|---|
| NaCl | 98 mM | 5,72 g/l |
| KCI | 6 mM | 0,45 g/l |
| NaH₂PO₄ | 2,5 mM | 0,3 g/l |
| Pyruvate de Na | 5 mM | 0,55 g/l |
| Fumarate de Na | 5 mM | 0,58 g/l |
| Glutamate de Na | 5 mM | 0,84 g/l |
| CaCl₂ | 1.5 mM | 0.22 g/l |
| | | |
| MgCl₂ | 1 mM | 0.20 g/l |
| HEPES | 25 mM | 6.07 g/l |
| Glucose | 11.5 mM | 2.07 g/l |
| Glutamine | 2 mM | 0.22 g/l |
| STI | | 0.1 g/l |

Pour l'expérience, le milieu est éliminé et les cellules sont lavées à deux reprises à l'aide de 1 ml de tampon. Le milieu réactionnel (500 µl) contenant l'agoniste marqué est ajouté dans chaque puits en présence ou en l'absence de l'agoniste homologue froid et les plaques sont incubées pendant une heure à 37 °C.

La réaction est arrêtée par aspiration du volume réactionnel et les puits sont rincés à deux reprises à l'aide de 1 mL de tampon BindH à 20% de BSA.

Les cellules sont lysées à l'aide de 500 µl de soude 1 N pendant 20 minutes à température ambiante et la radioactivité associée au lysat est évaluée à l'aide d'un compteur gamma.

### 2) Résultats:

Les peptides ainsi identifiés ont été testés selon les protocoles décrits ci-dessus.. Les résultats obtenus pour les peptides 1 à 6 sont repris dans le tableau ci-après.

| **Exemple** | **Test de liaison avec** | **Résultats** |
|---|---|---|
| ¹ | membranes de cervelle de cobaye membranes de différents organes du rat Cellules HeLa Cellules Jurkat T | positif (IC₅₀=100µM) négatif positif (IC₅₀=100µM) négatif |
| 2 | membranes de cervelle de cobaye membranes de différents organes du rat Cellules HeLa Cellules Jurkat T | positif (IC₅₀=100µM) positif (IC₅₀=100µM) positif (IC₅₀=100µM) positif (IC₅₀=100µm) |
| ³ | membranes de cervelle de cobaye membranes du cerveau/du foie et de différents organes du rat Cellules HeLa Cellules Jurkat T | Positif (IC₅₀=100µM) positif (IC₅₀=100µM) positif (IC₅₀=2µM) positif (IC₅₀=50µM) |
| 4 | membranes de cervelle de cobaye membranes de différents organes du rat Cellules HeLa Cellues Jurkat T | positif (IC₅₀=100µM) négatif positif (IC₅₀=100µM) positif (IC₅₀=100µM) |
| 5 | membranes de cervelle de cobaye membranes de cervelle de rat membranes de différents organes du rat Cellules Jurkat T | positif (IC₅₀=100µM) positif (IC₅₀=90µM) positif (IC₅₀=100µM) négatif |
| 6 | membranes de cervelle de cobaye membranes de cervelle de rat membranes de différents organes du rat Cellules Jurkat T | positif (IC₅₀=100µM) positif (IC₅₀=90µM) positif (IC₅₀=100µM) négatif |

Outre les peptides 1 à 6, les peptides suivants ont été obtenus et testés sur des membranes de cerveau de cobaye :

| Exemple | IC₅₀ (µM) | Exemple | IC₅₀ (µM) |
|---|---|---|---|
| 7 | 0,43 | 16 | 0,08 |
| 8 | 4,2 | 17 | 0,68 |
| 9 | 0,22 | 18 | 0,8 |
| 10 | 0,03 | 19 | 0,7 |
| 11 | 0,27 | 20 | 0,35 |
| 12 | 0,02 | 21 | 0,16 |
| 13 | 0,09 | 22 | 2,8 |
| 14 | 0,86 | 23 | 1,3 |
| 15 | 0,36 | 24 | 0,23 |
| 25 | 0,31 | 48 | 1,47 |
| 26 | 0,7 à 22 | 49 | 0,33 |
| 27 | 0,68 | 50 | 1 |
| 28 | 0,4 | 51 | 0,14 |
| 29 | 0,01 | 52 | 7 |
| 30 | 27 | 53 | 0,01 à 1 |
| 31 | 0,02 à 2 | 54 | 0,3 |
| 32 | 0,2 | 55 | 0,004 à 1 |
| 33 | 0,1 | 56 | 0,73 |
| 34 | 1 | 57 | 0,06 |
| 35 | 5,6 | 58 | 0,86 |
| 36 | 2,9 | 59 | 0,41 |
| 37 | 0,8 | 60 | < 0,1 à 4 |
| 38 | 0,5 | 61 | 1,11 |
| 39 | 0,4 | 62 | 0,67 |
| 40 | 0,13 | 63 | 0,02 |
| 41 | 0,25 | 64 | 0,001 |
| 42 | 0,56 | 65 | 4 |
| 43 | 0,05 | 66 | 0,002 |
| 44 | 0,1 | 67 | 0,16 |
| 45 | 0,7 | 68 | 0,025 |
| 46 | 0,1 | 69 | 0,037 |
| 47 | 0,3 | | |

## Revendications

1. Méthode d'identification de précurseurs d'un peptide comprenant une extrémité C-terminale amidée, comprenant les étapes suivantes :
- Extraction de séquences automatisées d'une source publique ;
- Analyse des fichiers et importation des données dans ORACLE^{™},
- Sélection des séquences à partir d'un sous-ensemble ou à partir de la banque de données toute entière ;
- Recherche des précurseurs d'un peptide comprenant une extrémité C-terminale amidée utilisant la séquence Y1-Y2-Y3-Y4-Y5, dans laquelle Y1 est une séquence nucléotidique de 1 à 12 nucléotides
ou Y1 est supprimée, Y2 est un codon pour Gly, Y3 et Y4 indépendamment sont des codons pour Arg ou Lys et Y5 est une séquence nucléotidique de 1 à 21 nucléotides ou Y5 est supprimé ;
- Vérification de l'absence de tout codon d'ARRET entre le codon AUG indiquant le début du cadre de lecture et le motif recherché ; et identification de telles séquences ;
- Vérification de ce que, si on le trouve, le motif est un cadre de lecture ouvert du type séquence consensus Kozak ;
- Comparaison de l'environnement de l'emplacement du motif à celui requis par recherche de structures secondaires requises autour d'un site de maturation telle que la proximité d'hélices alpha ou de feuilles bêta ;
- Vérification de la présence d'une similarité des séquences et d'autres séquences connues.

## Claims

1. Method for identifying precursors of a peptide comprising an amidated C-terminal end, comprising the following steps:
- Extraction of automated sequences from a public source;
- Analyse files and import data in ORACLE^{™}
- Select sequences from a sub-set or from the entire database;
- Search for precursors of a peptide comprising an amidated C-terminal end using the sequence Y1-Y2-Y3-Y4-Y5, in which Y1 is a nucleotide sequence of 1 to 12 nucleotides or Y1 is suppressed, Y2 is a codon for Gly, Y3 and Y4 independently are codons for Arg or Lys and Y5 is a nucleotide sequence of 1 to 21 nucleotides or Y5 is suppressed.
- Verification of the absence of any STOP codon between the AUG codon indicating the start of the reading frame and the sought motif; and identification of such sequences;
- Verification that when found, the motif is an open reading frame of Kozak consensus sequence type;
- Comparison of the environment of the motif location with the one required by searching secondary structures required around a maturation site such as the proximity of alpha-helices or beta-sheets;
- Verification for similarity of the sequences and other known sequences

## Patentansprüche

1. Verfahren zur Identifizierung von Vorläufern eines Peptids mit einem amidierten C-terminalen Ende, umfassend die folgenden Schritte:
- Extraktion von automatisierten Sequenzen aus einer öffentlichen Quelle;
- Analyse der Dateien und Import der Daten in ORACLE^{™};
- Selektion der Sequenzen aus einer Untermenge oder aus der gesamten Datenbank;
- Suche der Vorläufer eines Peptids mit einem amidierten C-terminalen Ende unter Verwendung der Sequenz Y1-Y2-Y3-Y4-Y5, in der Y1 eine Nucleotidsequenz von 1 bis 12 Nucleotiden ist oder Y1 entfällt, Y2 ein Codon für Gly ist, Y3 und Y4 unabhängig voneinander Codons für Arg oder Lys sind und Y5 eine Nucleotidsequenz von 1 bis 21 Nucleotiden ist oder Y5 entfällt;
- Überprüfung der Abwesenheit jedes STOP-Codons zwischen dem den Anfang des Leserahmens angebenden Codon AUG und dem gesuchten Motiv; und Identifizierung solcher Sequenzen;
- Überprüfung, ob das Motiv, wenn man es findet, ein offener Leserahmen vom Typ Kozak-Konsensus-Sequenz ist;
- Vergleich der Umgebung des Standorts des Motivs mit derjenigen, die erforderlich ist, durch Suche nach erforderlichen sekundären Strukturen um eine Reifungsstelle herum wie die Nähe von alpha-Helices oder beta-Faltblättern;
Überprüfung des Vorliegens einer Ähnlichkeit der Sequenzen und anderer bekannter Sequenzen.
